(19) Europäisches Patentamt
European Patent Office
Office européen des brevets

(11) Veröffentlichungsnummer: **0 381 086 A1**

(12) # EUROPÄISCHE PATENTANMELDUNG

(21) Anmeldenummer: **90101635.2**

(22) Anmeldetag: **27.01.90**

(51) Int. Cl.5: **C07C 43/12, C07C 41/36, C08G 65/30**

(30) Priorität: **31.01.89 DE 3902803**

(43) Veröffentlichungstag der Anmeldung: **08.08.90 Patentblatt 90/32**

(84) Benannte Vertragsstaaten: **AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Gries, Thomas, Dr.**
**Johannesallee 18**
**D-6230 Frankfurt am Main 80(DE)**

(54) **Verfahren zur Reinigung und Stabilisierung von Perfluorpolyethern.**

(57) Die Erfindung betrifft ein Verfahren zur Stabilisierung und Reinigung von Perfluorpolyethern der allgemeinen Formeln (I) oder (II)

$$(I)\qquad R[CF_2O\underset{CF_3}{CF}]_n\,[\underset{CF_3}{CF}OCF_2]_m\,R'$$

worin
$R$ = -F oder $-C_aF_{2a+1}$
$R'$ = -F oder $-C_bF_{2b+1}$
n,m = 0,1, ... oder 12, n gleich oder ungleich m, $1 \leq n+m \leq 15$
a,b = 1,2, ... oder 10, a gleich oder ungleich b
ist
(II) $R''O[C_3F_6O]_c\,[C_2F_4O]_d\,[CFXO]_e\,R'''$
worin
$R''$ = $-C_fF_{2f+1}$
$R'''$ = $-C_gF_{2g+1}$
X = -F oder $-CF_3$
c,d,e = ganze Zahlen, derart daß das Molekulargewicht des Perfluorpolyethers zwischen 200 und 2000 liegt, wobei c, d und e gleich oder ungleich sein können und die Einheiten $[C_3F_6O]_c$, $[C_2F_4O]_d$, und $[CFXO]_e$ willkürlich entlang der Molekülkette des Perfluorpolyethers verteilt sein können
oder von Gemischen derartiger Perfluorpolyether. Das Verfahren besteht darin, daß man die Perfluorpolyether oder deren Gemische auf 150-360 °C in Gegenwart eines Katalysators erhitzt, der ein oder mehrere Elemente der Gruppen IA, IIA, IIIA, IVA oder IVB oder deren Verbindungen enthält.

EP 0 381 086 A1

## Verfahren zur Reinigung und Stabilisierung von Perfluorpolyethern

Die Erfindung betrifft ein Verfahren zur Stabilisierung und Reinigung von Perfluorpolyethern der allgemeinen Formeln (I) oder (II)

$$\text{(I)} \qquad R[CF_2O\underset{CF_3}{\underset{|}{CF}}]_n\,[\underset{CF_3}{\underset{|}{CF}}OCF_2]_m\,R'$$

worin
$R = -F$ oder $-C_aF_{2a+1}$
$R' = -F$ oder $-C_bF_{2b+1}$
n,m = 0,1, ... oder 12, n gleich oder ungleich m, $1 \leq n+m \leq 15$
a,b = 1,2, ... oder 10, a gleich oder ungleich b
ist
(II) $R''O[C_3F_6O]_c\,[C_2F_4O]_d\,[CFXO]_e\,R'''$
worin
$R'' = -C_fF_{2f+1}$
$R''' = -C_gF_{2g+1}$
X = -F oder $-CF_3$
c,d,e = ganze Zahlen, derart daß das Molekulargewicht des Perfluorpolyethers zwischen 200 und 2000 liegt, wobei c, d und e gleich oder ungleich sein können und die Einheiten $[C_3F_6O]_c$, $[C_2F_4O]_d$, und $[CFXO]_e$ willkürlich entlang der Molekülkette des Perfluorpolyethers verteilt sein können
oder von Gemischen derartiger Perfluorpolyether.

Die Herstellung dieser Perfluorpolyether ist bekannt aus US-PS 3 665 041, US-PS 3 985 810 und JP-OS Sho 58-103 334 (Anmeldungsnummer Sho 56-199 999). Perfluorpolyether enthalten je nach Typ und Herstellverfahren unterschiedliche Mengen verschiedener Nebenprodukte, die bereits in Spuren von 0,1 Gew.-% durch Nachsäuern, d.h. langsames Abgeben saurer und korrosiver Gase, und durch unangenehmen Geruch den inerten Charakter und die Anwendung der - in reiner Form neutralen und stabilen - Perfluorpolyether beeinträchtigen.

Diese Nebenprodukte sind im allgemeinen toxische oder reaktive fluorierte Verbindungen, die außerdem noch Wasserstoff enthalten können, und sie bilden unter den bei der Anwendung von Perfluorpolyethern herrschenden Bedingungen ebenfalls toxische oder reaktive, saure Produkte niedrigeren Molekulargewichtes, wie z.B. Carbonylfluoride, perfluorierte Carbonsäuren, Fluoralkene oder Fluorwasserstoff.

Für die wichtige Anwendung der Perfluorpolyether als Flüssig- oder Dampf-Phasen-Wärmeüberträger, (z.B bei dem in US-PS 3 866 307, US-PS 3 904 102 und US-PS 4 721 578 beschriebenen Kondensationslöten von elektronischen Bauteilen) oder als Testflüssigkeit für die Prüfung elektronischer Bauteile (z.B. "Thermal Shock Test", "Gross Leak Test" und "Burn in Test", wie in EP-A-0 203 348 beschrieben), ist die Abwesenheit der genannten Verunreinigungen Bedingung,

1. um die Kompatibilität mit den Werkstoffen von Bauteilen und Apparaten zu erhalten,
2. um eine Gefährdung des Anwenders und der Umwelt durch giftige Produkte zu vermeiden, und
3. um eine Verwendung über einen langen Zeitraum zu ermöglichen.

Es ist daher wünschenswert, eine wirksame und rationelle technische Reinigungsmethode zu finden, um Perfluorpolyether in eine wirklich inerte Form überführen zu können, die diese Kriterien erfüllt.

Eine Reinigung bestimmter Perfluorpolyether ist beispielsweise in der EP-PS 0 158 446 beschrieben. Dieses Verfahren ist jedoch auf Perfluorpolyether beschränkt, die bei 150-200 °C entgast werden können, ohne dabei selbst zu verdampfen. Es erfordert darüberhinaus die Bestrahlung mit UV-Licht und den technisch aufwendigen Umgang mit aggressiven oder teuren Gasen wie Fluor, Chlor oder Sauerstoff.

Die in JP-OS Sho 58-103 334 (siehe oben) beschriebene Aufarbeitung von Perfluorpolyethern durch Trocknung und Destillation ergibt keine reinen Produkte, wie die dort angegebenen Wasserstoffanalysen beweisen, und führt zur Bildung von fluorierten Carbonsäuren und Fluorwasserstoff.

Es wurde nun überraschenderweise gefunden, daß man die oben definierten Perfluorpolyether der Formeln (I) und (II), z.B. die gemäß Sho 58-103 334 hergestellten, reinigen kann, indem man die Nebenprodukte bei hohen Temperaturen mit Hilfe eines Katalysators thermisch zersetzt.

Gegenstand der Erfindung ist ein Verfahren zur Stabilisierung und Reinigung von Perfluorpolyethern der allgemeinen Formeln (I) oder (II)

$$(I) \quad R[CF_2OCF(CF_3)]_n [CF(CF_3)OCF_2]_m R'$$

worin
R = -F oder $C_aF_{2a+1}$
R' = -F oder $-C_bF_{2b+1}$
n,m = 0,1, ... oder 12, n gleich oder ungleich m, $1 \leq n+m \leq 15$
a,b = 1,2, ... oder 10, a gleich oder ungleich b
ist
(II) $R''O[C_3F_6O]_c [C_2F_4O]_d [CFXO]_e R'''$
worin
R'' = $-C_fF_{2f+1}$
R''' = $-C_gF_{2g+1}$
X = -F oder $-CF_3$
c,d,e = ganze Zahlen, derart daß das Molekulargewicht des Perfluorpolyethers zwischen 200 und 2000 liegt, c, d und e gleich oder ungleich sein können und die Einheiten $[C_3F_6O]_c$, $[C_2F_4O]_d$, und $[CFXO]_e$ willkürlich entlang der Molekülkette des Perfluorpolyethers verteilt sein können
oder von Gemischen derartiger Perfluorpolyether, dadurch gekennzeichnet, daß man die Perfluorpolyether oder deren Gemische auf 150-360 °C in Gegenwart eines Katalysators erhitzt, der ein oder mehrere Elemente der Gruppen IA, IIA, IIIA, IVA oder IVB des Periodensystems oder deren Verbindungen enthält.

Besonders wichtig sind diejenigen Perfluorpolyether der Formel (I), die eine der Merkmalskombinationen 1 bis 3 haben:

1. R = R' = $-C_2F_5$
n = 1, 2, 3 oder 4; m = 0, 1, 2, 3 oder 4
n gleich oder ungleich m

2. R = $-C_2F_5$, R' = $-C_7F_{15}$
n = 1, 2, 3 oder 4; m = 0

3. R = $-C_2F_5$, R' = -F
n = 1, 2, ... oder 9; m = 0

Weiter sind wichtig diejenigen Perfluorpolyether der Forme (II), die eine der Merkmalskombinationen 4 bis 7 haben:

4. R'' = R''' = $-CF_3$; X = -F
c = 0; d,e ≠ 0

5. R'' = R''' = $-CF_3$; $-C_2F_5$ oder $-C_3F_7$
X = -F oder $-CF_3$
c, e ≠ 0; d = 0

6. R'' = $-C_3F_7$ oder $-C_4F_9$
R''' = $-C_2F_5$ oder $-C_3F_7$
c = e = 0; d ≠ 0

7. R'' = $-CF_3$, $-C_2F_5$ oder $-C_3F_7$
R''' = $-CF_3$, $-C_2F_5$ oder $-C_3F_7$
c ≠ 0; d = e = 0

Nach dem erfindungsgemäßen Verfahren lassen sich beliebige Gemische aus mehreren Perfluorpolyethern der Formeln (I) oder (II) reinigen und stabilisieren, eine besonders wichtige Ausführungsform ist jedoch die Reinigung und Stabilisierung eines einzelnen Ethers, insbesondere eines solchen der Formel (I), der eine der Merkmalskombinationen 1., 2. oder 3. besitzt. Eine weitere besonders wichtige Ausführungsform ist die Reinigung und Stabilisierung eines 3-Komponentengemisches, das einen symmetrischen Ether der Formel

$$R[CF_2OCF(CF_3)]_n [CF(CF_3)OCF_2]_n R,$$

n = 1, 2, 3 oder 4,
einen anderen symmetrischen Ether der Formel

$$R'[CF_2O\underset{CF_3}{\underset{|}{C}}F]_m\ [\underset{CF_3}{\underset{|}{C}}FOCF_2]_m\ R',$$

m = 1, 2, 3 oder 4, wobei m ≠ n ist, und den korrespondierenden unsymmetrischen Ether der Formel

$$R[CF_2O\underset{CF_3}{\underset{|}{C}}F]_n\ [\underset{CF_3}{\underset{|}{C}}FOCF_2]_m\ R'$$

enthält, wobei für R und R′ wieder die obigen allgemeinen Definitionen gelten (R gleich oder ungleich R′).

Solche 3-Komponentengemische entstehen bei der elektrolytischen Decarboxylierung eines Gemisches von zwei Perfluorcarbonsäuren der Formeln

$$R[CF_2\text{-}O\text{-}\underset{CF_3}{\underset{|}{C}}F]_nCOOH \text{ und } R'[CF_2\text{-}O\text{-}\underset{CF_3}{\underset{|}{C}}F]_mCOOH$$

(vgl. JP-OS Sho 58-103 334).

Eine weitere besonders wichtige Ausführungsform ist die Reinigung und Stabilisierung eines 2-Komponentengemisches, das einen symmetrischen Ether der Formel R[ ]$_n$ [ ]$_n$ R und einen unsymmetrischen Ether der Formel R[ ]$_n$ R′ enthält, wobei n = 1, 2, 3 oder 4 ist und [ ] für

$$[CF_2O\underset{CF_3}{\underset{|}{C}}F]$$

steht.

Vorzugsweise werden die Perfluorpolyether oder deren Gemische auf 200-360 °C erhitzt, insbesondere auf 270-330 °C.

Als Katalysatoren verwendet man Stoffe, die ein oder mehrere Elemente der Gruppen IA (Li bis Cs), IIA (Be bis Ba), IIIA (B bis Tl), IVA (C bis Pb) oder IVB (Ti bis Hf) oder deren Verbindungen enthalten. Geeignete Verbindungen sind beispielsweise die Oxide, Carbonate, Halogenide und Hydroxide. Vorzugsweise enthalten die Katalysatoren Na, K, Cs, Mg, Ca, Ba, Ti, B, Al, C oder Si. Besonders geeignete Katalysatoren sind Aktivkohle, Calciumoxid, Titandioxid, Kaliumfluorid und Aluminiumoxid.

Das erfindungsgemäße Verfahren läßt sich prinzipiell in der Flüssigphase in einem einfachen Rührkessel durchführen oder durch Überleiten der Flüssigkeit über einen Festbettkatalysator. Es ist jedoch von Vorteil, die zu reinigenden Perfluorpolyether zu verdampfen und gasförmig über einem Festbettkatalysator in einem Rohrreaktor zu leiten, d.h. die Thermolyse der Verunreinigungen als Gas-Feststoff-Reaktion auszuführen. Bei hochsiedenden Perfluorpolyethern kann durch Erhitzen im Vakuum ein Verdampfen erzielt werden. Dabei ist es unkritisch, in welcher räumlichen Anordnung die Reaktion erfolgt. Vorzugsweise wird jedoch der gasförmige Perfluorpolyether von unten durch einen senkrecht angeordneten Rohrreaktor geleitet. Die gasförmigen Reaktionsprodukte verlassen den Reaktor oben und werden anschließend kondensiert.

Die erfindungsgemäß gereinigten Perfluorpolyether sind thermisch stabil und erzeugen bei ihrem Einsatz keine Zersetzungsprodukte. Sie können für Anwendungen, bei denen eine besonders hohe Reinheit erforderlich ist, anschließend an das erfindungsgemäße Verfahren durch übliche Trennverfahren, z.B. durch Wäsche mit Wasser oder wäßrigen Lösungen von Alkali- oder Erdalkalihydroxiden, oder durch Feststoffadsorption (z.B. mit Alkali- oder Erdalkalihydroxiden oder -carbonaten, Aktivkohle, Kieselgel oder $Al_2O_3$), ggf. noch ergänzt durch eine anschließende Destillation, in einer einheitlichen Reinheit von 99,9 % (GC) erhalten werden.

Wenn man in der Gasphase arbeitet und eine zusätzliche Reinigung an das erfindungsgemäße Verfahren anschließen will, so ist es vorteilhaft, die den Reaktor oben verlassenden gasförmigen Reaktionsprodukte durch Einleiten in wäßrige KOH- oder NaOH-Lösungen zu kondensieren, wobei sich die gereinigten Perfluorpolyether als schwere Phase unten absetzen. Dadurch ist eine kontinuierliche Arbeitsweise möglich. Man kann jedoch auch eine Blasensäule oder einen Schlaufenreaktor verwenden.

Die nach diesem Verfahren gereinigten Produkte sind selbst in Gegenwart von Metallen und von Materialien, die beim Löten von elektronischen Bauteilen verwendet werden, bei Siedetemperatur auch über einen Zeitraum von 1000 Stunden beständig. Auch bei Temperaturen von 350 °C (z.B. durch Überhitzung) und in Gegenwart der genannten Werkstoffe sind die Perfluorpolyether stabil und bilden keine giftigen Zersetzungsprodukte. Besonders hervorzuheben ist, daß die nach dem erfindungsgemäßen Verfahren gereinigten Perfluorpolyether, im Gegensatz zu den nicht auf diese Weise gereinigten, unter thermischer Belastung kein Perfluorisobuten bilden, wlches als besonders toxisches Zersetzungsprodukt die Anwendung der Perfluorpolyether beeinträchtigt. Durch die erfindungsgemäße Reinigung wird das Lösungsvermögen der Perfluorpolyether für die beim Kondensationslöten elektronischer Bauteile verwendeten Lötpasten-Flußmittel erheblich reduziert. Die erfindungsgemäß stabilisierten Perfluorpolyether erfüllen die einleitend genannten Kriterien und eignen sich insbesondere für die Anwendung beim Kondensationslöten.

Das erfindungsgemäße Verfahren eignet sich auch für die Aufarbeitung bereits benutzter und dadurch verunreinigter Perfluorpolyether.

Die in den folgenden Beispielen genannten Prozente sind Gewichtsprozente, falls nichts anderes angegeben wird oder sich aus dem Zusammenhang ergibt.

## Vergleichsbeispiel

4109 g eines Rohproduktes von

$$\underline{1} = (C_2F_5(CF_2OCF)_2)_2$$
$$CF_3$$

(Gehalt 86,5 %), hergestellt durch Kolbe-Elektrolyse von

$$C_2F_5(CF_2OCF)_2COOH,$$
$$CF_3$$

wurden mit 800 ml 2,7 %iger Natronlauge 5 h lang gerührt. Nach Abtrennen und Waschen mit Wasser wurden 3880 g erhalten. Die Vakuumdestillation in einer 1,8 m langen Füllkörperkolonne mit 50 Trennstufen ergab bei einem Rücklaufverhältnis von 20:1 eine Ausbeute von 2235 g 1 in einer maximalen Reinheit von 99,8 % (GC). Die Substanz besaß einen pH-Wert von 1 und reagierte korrosiv.

## Beispiel 1

359 g

$$\underline{2} = (C_2F_5(CF_2OCF)_3)_2,$$
$$CF_3$$

mit einem pH-Wert von 1 wurden mit 3,6 g Aktivkohle 20 h lang bei 200 °C gerührt. Nach dem Abfiltrieren wurde mit 10 %iger Natronlauge gewaschen. Der pH-Wert war anschließend neutral und die Substanz setzte auch beim Erhitzen auf 300 °C keine sauren Gase frei. Ausbeute: 294 g.

## Beispiel 2

807 g Rohprodukt 1 wurden während 1,5 h bei 280 °C durch ein waagrecht aufgebautes Tonrohr, das mit 200 g Aktivkohle gefüllt war, gepumpt und anschließend in einer NaOH-Wäsche aufgefangen. Nachwaschen mit Wasser ergab 604 g gereinigtes 1, welches nicht mehr säuerte.

**Beispiel 3**

2410 g Rohprodukt 1

$$(\text{Gehalt } 90{,}9\ \%;\ 7{,}2\ \%\ C_2F_5(CF_2O\underset{CF_3}{\underset{|}{C}}F)_2OCH_3$$

$$= 3,\ \text{und } 1{,}5\ \%\ C_2F_5(CF_2O\underset{CF_3}{\underset{|}{C}}F)_2COOCH_3 = 4)$$

wurden gasförmig bei 320 °C durch ein mit Aktivkohle gefülltes und beheiztes Stahlrohr geführt und in NaOH-Lösung aufgefangen. Nachwaschen mit Wasser ergab 2151 g 1. Die Substanzen 3 und 4 waren vollständig entfernt. Die Probe säuerte auch nach mehreren Tagen nicht nach. Ausbeute: 97 %.

**Beispiel 4**

2337 g 1, welches 3,5 % 3 enthielt, wurden während 4,5 h gasförmig durch ein 1 m langes beheiztes Stahlrohr mit der bereits in Beispiel 3 benutzten Aktivkohle bei 305 °C geleitet und in einer NaOH-Wäsche kondensiert. Nach Abtrennen und Waschen mit Wasser wurden 2050 g 1 aufgefangen. Gemäß GC-Analyse war 3 nicht mehr in dem Produkt enthalten. Die anschließende Vakuumdestillation ergab 1990 g 1 mit einer Reinheit von mehr als 99,9 % (GC). Ausbeute: 88,3 %.

**Beispiel 5**

470 g saures Rohprodukt

$$(C_2F_5(CF_2O\underset{CF_3}{\underset{|}{C}}F)_4)_2 = 5$$

wurden bei 300 °C in flüssiger Phase durch ein 25 cm langes beheiztes Rohr gepumpt, in dem sich 8,4 ,g einer 1:3 Mischung (Gewichtsverhältnis) von Kaliumfluorid und Aktivkohle befanden. Nach dem Waschen mit KOH-Lösung wurden 365 g 5 mit einem Siedepunkt von 102 °C bei 1 mbar erhalten. Der pH-Wert war neutral.

**Beispiel 6**

In einem 25 cm langen Rohrreaktor wurden bei 280 bis 350 °C verschiedene Katalysatoren zur Reinigung und Stabilisierung eines Rohproduktes der Zusammensetzung

$$(C_3F_7O\underset{CF_3}{\underset{|}{C}}F)_2 \quad = 6 \quad 97{,}2\ \%$$

$$C_3F_7O\underset{CF_3}{\underset{|}{C}}F\text{-}COOCH_3 \quad = 7 \quad 0{,}8\ \%$$

$$C_3F_7O\underset{CF_3}{\underset{|}{C}}F\text{-}OCH_3 \quad = 8 \quad 1{,}4\ \%$$

verwendet. Die Resultate waren wie folgt:

| Katalysator | Durchsatz g/h | Masse Edukt / Masse Kat. | Endgehalt (% GC) an 7 | 8 |
|---|---|---|---|---|
| Aktivkohle | 20 | 9 | - | 0,01 |
| KF/Aktivkohle | 21 | 12,9 | - | - |
| Kieselgel | 22 | 8,0 | - | 0,04 |
| Aluminiumoxid | 18 | 6,9 | - | - |

| Katalysator | Durchsatz g/h | Masse Edukt / Masse Kat. | Endgehalt (% GC) an 7 | 8 |
|---|---|---|---|---|
| Aluminiumoxid | 9 | 62,6 | - | - |
| KF/Aluminiumoxid | 24 | 6,1 | - | - |
| Molekularsieb 4 Å | 18 | 5,4 | 0,02 | 0,02 |
| $TiO_2$/Aktivkohle | 18 | 8,6 | - | 0,03 |
| Calciumcarbonat | 17 | 7,7 | - | 0,02 |

**Beispiel 7**

177 g eines sauren Rohgemisches von Perfluorpolyethern (46 %

$C_3F_7OCF-(CFOCF_2)_3C_2F_5$ = 9, 20 % 6 und 27 % $(C_2F_5(CF_2OCF)_3)_2$
$\quad\quad\quad CF_3 \quad CF_3 \quad\quad\quad\quad\quad\quad\quad\quad\quad\quad\quad\quad\quad\quad\quad\quad CF_3$

= 10

wurden bei 340° C durch einen Stahlreaktor (Länge = 25 cm, Durchmesser = 1 cm) mit 3,4 g Aktivkohle geleitet und anschließend mit KOH-Lösung gewaschen. Die Substanz säuerte danach nicht mehr und ihr pH-Wert war neutral.

**Beispiel 8**

Durch ein mit 15 g Aluminiumoxid gefülltes Stahlrohr wurden bei 300° C während 13 h 126 g 6 geleitet, worin 1 % 8, 3 % 7 und 4 %

$C_3F_7OCFCOOH$ = 11
$\quad\quad\quad CF_3$

enthalten waren. Nach dem Kondensieren waren 7, 8 und 11 vollständig entfernt.

**Beispiel 9**

Während 370 h wurden 357 kg 1, das 1,0 % 4 und 4,1 % 3 enthielt, nach Verdampfen durch ein 1 m langes beheiztes Stahlrohr, welches mit 1,2 kg Aluminiumoxid gefüllt war, bei 250 bis 300 °C geleitet und in einer KOH-Wäsche kondensiert. Dabei wurden 3 und 4 selektiv zersetzt, so daß ihr Gehalt im gereinigten Produkt unter 0,1 % lag.

**Beispiel 10**

Es wurde die Löslichkeit eines Flußmittels für Lötpasten, das beim Kondensationslöten elektronischer Bauteile benutzt wird, a) in einem Rohprodukt von 1 und b) in einem durch Thermolyse in der Gasphase in Gegenwart eines $Al_2O_3$-Katalysators und anschließende Wäsche mit wäßriger NaOH-Lösung gereinigten 1 jeweils bei 20 und 220 °C untersucht. Die Bestimmung der Löslichkeit des Flußmittels erfolgte gaschromatographisch nach Silylierung der Proben. Als Referenz diente eine definierte Lösung des Flußmittels in Trichlortrifluorethan.

A. 40 g Rohprodukt 1 wurden mit 0,4 g Flußmittel 3 h bei 20 °C gerührt und anschließend filtriert.

B. 40 g Rohprodukt 1 wurden mit 0,4 g Flußmittel 3 h bei 220 °C unter Rückfluß gekocht und anschließend filtriert.

C. 40 g gereinigtes 1 wurden mit 0,4 g Flußmittel 3 h bei 20 °C gerührt und anschließend filtriert.

D. 40 g gereinigtes 1 wurden mit 0,4 g Flußmittel 3 h bei 220 °C unter Rückfluß gekocht und anschließend filtriert.

Ergebnis:

Der Gehalt an Flußmittel war wie folgt:

A: 0,3 %
B: 0,3 %
C: 0,1 %
D: 0,1 %

**Beispiel 11**

Vier 100 ml Glasampullen wurden mit je 20 ml 1 gefüllt, das zuvor wie in Beispiel 10, jedoch mit KF/Aktivkohle statt $Al_2O_3$, gereinigt und stabilisiert worden war. Zu drei Proben wurden 2,5 g Lötpaste gegeben, wie sie beim Kondensationslöten benutzt wird. Zwei Proben enthielten zusätzlich ein V4A-Stahlblech. Die abgeschmolzenen Ampullen wurden 1000 h lang bei 220 °C gelagert.

Nach Abkühlen auf -78 °C wurden die Ampullen am oberen Ende aufgeschnitten und durch eine Septum-Verschraubung wieder verschlossen. Nach dem Erwärmen der Ampullen wurden mittels gasdichter Spritze Proben aus dem Gasraum entnommen und gaschromatographisch untersucht. Neben einem Übersichtschromatogramm wurde der Gehalt an Perfluorisobuten quantitativ bestimmt.

Ergebnis:

Keine der Proben wies Zersetzungsprodukte von 1 auf. In keiner der Proben war Perfluorisobuten enthalten (Nachweisgrenze 0,1 ppm).

**Beispiel 12**

Vier Glasampullen wurden wie in Beispiel 11 beschrieben mit 1, Lötpaste und Stahlblech gefüllt. Die Proben wurden dann 100 h bei 350 °C gelagert. Die Aufarbeitung und Analyse erfolgte wie in Beispiel 11 beschrieben.

Ergebnis:

Keine der Proben wies Zersetzungsprodukte von $\underline{1}$ auf. In keiner der Proben war Perfluorisobuten enthalten.

## Ansprüche

1. Verfahren zur Stabilisierung und Reinigung von Perfluorpolyethern der allgemeinen Formeln (I) oder (II)

$$(I) \qquad R[CF_2O\underset{CF_3}{CF}]_n\ [\underset{CF_3}{CF}OCF_2]_m\ R'$$

worin
R = -F oder $-C_aF_{2a+1}$
R′ = -F oder $-C_bF_{2b+1}$
n,m = 0,1, ... oder 12, n gleich oder ungleich m, $1 \leq n+m \leq 15$
a,b = 1,2, ... oder 10, a gleich oder ungleich b
ist
(II) $R''O[C_3F_6O]_c\,[C_2F_4O]_d\,[CFXO]_e\,R'''$
worin
$R'' = -C_fF_{2f+1}$
$R''' = -C_gF_{2g+1}$
X = -F oder $-CF_3$
c,d,e = ganze Zahlen, derart daß das Molekulargewicht des Perfluorpolyethers zwischen 200 und 2000 liegt, wobei c, d und e gleich oder ungleich sein können und die Einheiten $[C_3F_6O]_c$, $[C_2F_4O]_d$, und $[CFXO]_e$ willkürlich entlang der Molekülkette des Perfluorpolyethers verteilt sein können oder von Gemischen derartiger Perfluorpolyether, dadurch gekennzeichnet, daß man die Perfluorpolyether oder deren Gemische auf 150-360 °C in Gegenwart eines Katalysators erhitzt, der ein oder mehrere Elemente der Gruppen IA, IIA, IIIA, IVA oder IVB des Periodensystems oder deren Verbindungen enthält.

2. Verfahren nach Anspruch 1, dadurch gekennzeichnet, daß man einen Perfluorpolyether der Formel (I) einsetzt, für den R = R′ = $-C_2F_5$, n = 1, 2, 3 oder 4 und m = 0, 1, 2, 3 oder 4 gilt, wobei n gleich oder ungleich m sein kann.

3. Verfahren nach Anspruch 1, dadurch gekennzeichnet, daß man einen Perfluorpolyether der Formel (I) einsetzt, für den R = $-C_2F_5$, R′ = $-C_7F_{15}$, n = 1, 2, 3 oder 4 und m = 0 gilt.

4. Verfahren nach Anspruch 1, dadurch gekennzeichnet, daß man einen Perfluorpolyether der Formel (I) einsetzt, für den R = $-C_2F_5$, R′ = -F, n = 1, 2, ... 8 oder 9 und m = 0 gilt.

5. Verfahren nach Anspruch 1, dadurch gekennzeichnet, daß man einen Perfluorpolyether der Formel (II) einsetzt, der eine der Merkmalskombinationen A, B, C oder D besitzt

A: $R'' = R''' = -CF_3$; X = -F
c = 0; d, e ≠ 0

B: $R'' = R''' = -CF_3$, $-C_2F_5$ oder $-C_3F_7$
X = -F oder $-CF_3$
c, e ≠ 0; d = 0

C: $R'' = -C_3F_7$ oder $-C_4F_9$
$R''' = -C_2F_5$ oder $-C_3F_7$
c = e = 0; d ≠ 0

D: $R'' = -CF_3$, $-C_2F_5$ oder $-C_3F_7$
$R''' = -CF_3$, $-C_2F_5$ oder $-C_3F_7$
c ≠ 0; d = e = 0

6. Verfahren nach Anspruch 1, dadurch gekennzeichnet, daß man ein Gemisch aus drei Perfluorpolyethern einsetzt, das einen symmetrischen Ether der Formel

$$R[CF_2O\underset{CF_3}{\underset{|}{C}}F]_n\ [\underset{CF_3}{\underset{|}{C}}FOCF_2]_n\ R,$$

n = 1, 2, 3 oder 4,
einen anderen symmetrischen Ether der Formel

$$R'[CF_2O\underset{CF_3}{\underset{|}{C}}F]_m\ [\underset{CF_3}{\underset{|}{C}}FOCF_2]_m\ R',$$

m = 1, 2, 3 oder 4,
wobei m ≠ n ist, und den korrespondierenden unsymmetrischen Ether der Formel

$$R[CF_2O\underset{CF_3}{\underset{|}{C}}F]_n\ [\underset{CF_3}{\underset{|}{C}}FOCF_2]_m\ R'$$

enthält.

7. Verfahren nach Anspruch 1, dadurch gekennzeichnet, daß man ein Gemisch aus zwei Perfluorpolyethern einsetzt, das einen symmetrischen Ether der Formel

$$R[CF_2O\underset{CF_3}{\underset{|}{C}}F]_n\ [\underset{CF_3}{\underset{|}{C}}FOCF_2]_n\ R$$

und einen unsymmetrischen Ether der Formel

$$R[CF_2O\underset{CF_3}{\underset{|}{C}}F]_n\ R'$$

enthält, wobei n = 1, 2, 3 oder 4 ist.

8. Verfahren nach mindestens einem der Ansprüche 1 bis 7, dadurch gekennzeichnet, daß man auf 200 bis 360 °C erhitzt.

9. Verfahren nach mindestens einem der Ansprüche 1 bis 7, dadurch gekennzeichnet, daß man auf 270 bis 330 °C erhitzt.

10. Verfahren nach mindestens einem der Ansprüche 1 bis 9, dadurch gekennzeichnet, daß man einen Katalysator verwendet, der ein oder mehrere der Elemente Na, K, Cs, Mg, Ca, Ba, Ti, B, Al, C oder Si enthält.

11. Verfahren nach mindestens einem der Ansprüche 1 bis 10, dadurch gekennzeichnet, daß man die Perfluorpolyether gasförmig über den Katalysator leitet.

Europäisches Patentamt

# EUROPÄISCHER RECHERCHENBERICHT

Nummer der Anmeldung

EP 90101635.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| X | US - A - 4 720 527 (GERARDO CAPORICCIO et al.) * Ansprüche 1,3 * -- | 1,8-10 | C 07 C 43/12<br>C 07 C 41/36<br>C 08 G 65/30 |
| D,X | US - A - 3 985 810 (SIGMAR-PETER VON HALASZ et al.) * Ansprüche 1,7-9 * -- | 1,8,9 | |
| D,A | EP - A1 - 0 158 446 (MONTEDISON S.P.A.) * Zusammenfassung * ---- | 1 | |

RECHERCHIERTE SACHGEBIETE (Int Cl 5)

C 07 C 43/00
C 07 C 41/00
C 08 G 65/00

Der vorliegende Recherchenbericht wurde für alle Patentansprüche erstellt

| Recherchenort | Abschlußdatum der Recherche | Prüfer |
|---|---|---|
| WIEN | 02-05-1990 | REIF |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82